# EUROPEAN PATENT APPLICATION

(11) **EP 1 048 302 A2**
(43) Date of publication of application: **02.11.2000**
(21) Application number: 00303348.7
(22) Date of filing: 20.04.2000
(51) Int. Cl.: A61K 51/04

(54) **Radiotracers for in vivo study of acetylcholinesterase and Alzheimer's disease**

(30) Priority: 30.04.1999 US 132113 P
(71) Applicant: Pfizer Products Inc., Groton, CT 06340-5146 (US)
(72) Inventor: Bencherif, Badreddine, Baltimore, Maryland 21287-0750 (US); Frost, James J., Baltimore, Maryland 21287-0750 (US); Dannals, Robert F., Baltimore, Maryland 21287-0750 (US); Musachio, John, Baltimore, Maryland 21287-0750 (US); Scheffel, Ursula, Baltimore, Maryland 21205-2195 (US); Villalobos, Anabella, Departement of Neuroscience, Groton, Connecticut 06340 (US)
(74) Representative: Simpson, Alison Elizabeth Fraser

(57) **Abstract**

Radio-labeled compounds of the formula 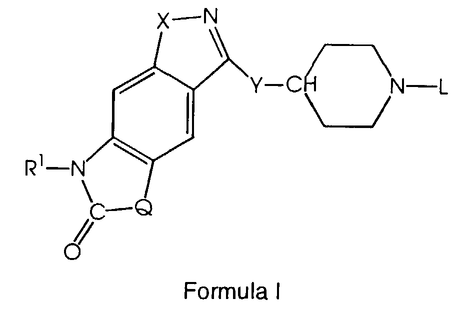 are useful as *in vivo* imaging agents for diagnosis of Alzheimer's disease.

## Description

### Background of the Invention

The present invention relates to methods of using radiolabeled heterocyclic-cyclic amine derivatives of the formula I below, and pharmaceutically acceptable salts of such compounds. The compounds of formula I are acetylcholinesterase (AChE) inhibitors and are useful as imaging agents for brain AChE in patients suffering from dementia and Alzheimer's disease. These agents are useful for early diagnosis of Alzheimer's disease and following the course of this illness.

Alzheimer's disease is associated with degeneration of cholinergic neurons in the basal forebrain that play a fundamental role in cognitive functions, including memory. Becker et al., *Drug Development Research*, 12, 163-195 (1988). As a result of such degeneration, patients suffering from the disease exhibit a marked reduction in acetylcholine synthesis, choline acetyltransferase activity, acetylcholinesterase activity and choline uptake.

The compounds of the present invention are potent inhibitors of AChE and bind strongly to the enzyme. Positron emission tomography (PET) and Single Photon Emission Computerized Tomography (SPECT) scanning techniques are useful to determine the distribution of the radiolabeled compound and the corresponding distribution of AChE.

Loss of cholinergic-mediated function and cholinergic markers in the brain is a characteristic of Alzheimer's disease (AD) that has been observed in numerous studies that show that patients with AD undergo a progressive, inexorable decline in cholinergic function.^{1,2} Furthermore, decreased cholinergic function may underlie the cognitive decline seen in AD patients.³ Attempts to study cholinergic function *in vivo* have involved development of radiopharmaceuticals for imaging acetylcholinesterase (AChE), high-affinity choline transporter on nerve endings and muscarinic and nicotinic receptors.⁴ Current therapeutic strategies often involve development of potent and selective inhibitors of AChE in an effort to increase cholinergic neurotransmission.^{5,6} Although such therapy could ultimately prove only palliative, as cholinergic neurons continue to decline through the course of AD, AChE itself could provide a very useful marker for several diagnostic and prognostic aspects of the disease. Specifically, a suitable radiolabeled AChE inhibitor may be used *in vivo* for 1) early diagnosis of AD, 2) elucidation of the progression of cholinergic loss in AD since patients with very early disease can be studied by position emission tomography (PET) throughout their illness, 3) establishment of possible correlation between cholinergic loss and memory test scores (e.g., MMSE scores) and 4) individualization of drug therapy with AChE inhibitors (e.g., patients with a small AChE decrement would be treated initially with a low drug dose and conversely).

A high-affinity, brain selective cholinergic marker, suitable for *in vivo* imaging in humans has not yet been developed; however, several carbon-11 AChE inhibitor analogs have been synthesized.⁴ A carbon-11 labeled analog of the AChE inhibitor tacrine, [¹¹C]MTHA, has been synthesized, but rodent and primate biodistribution studies failed to show co-localization of [¹¹C]MTHA binding sites with AChE.⁹ [¹¹C]Physostigmine distribution proved superimposable with that of AChE, and that compound as been used to visualize AChE *in vivo* (rats and primates) with PET.^{10, 11} However, brain AChE quantification and binding kinetics are not available for [¹¹C]physostigmine so that it is difficult to predict what effect the short half life of physostigmine will have on its suitability as a PET imaging agent. Another class of AChE inhibitors, the piperidyl esters, have been prepared in radiolabeled form.¹³ Although preliminary rodent biodistribution studies are encouraging, no PET data has yet been published.

The compound of Example 2 is an example of a class of AChE inhibitors which are new, highly potent and selective.¹² It has high affinity (IC₅₀ of 0.48n*M*) and unprecedented selectivity (9300:1 AChE relative to BChE) for AChE. A radiopharmaceutical based on compound of Example 2 would meet the necessary criteria for study of cholinergic neurotransmission *in vivo*, namely, high affinity and selectivity, high brain uptake, *in vivo* stability and ease of radiochemical synthesis.

### References

1. Davis, P. *Brain Res*. **1979**, *171*, 319-327.
2. Hardy, J.; Adolfsson, I.; Alafuzoff, G.; Bucht, J.; Marcusson, P.; Nyberg, E.; Perdahl, P.; Wester, P.; Windblad, B. *Neurochem. Int*., **1985**, 7, 545-563.
3. Kish, S. J.; Schut, L.; Simmons, J.; Gilbert, J.; Chang, L.; Rebbetoy, M. *J*. *Neurol*., *Neurosurg*., *and Psych*. **1988**, *51*, 544-548.
4. Maziere, M. *Pharmac. Ther*. **1995**, *66*, 83-101.
5. Vidaluc, J.; Calmel, F., Bigg, D.C.H.; Carilla, E.; Briley, M. *J. Med. Chem*. **1995**, *38*, 2969-2973.
6. Villalobos, A.; Butler, T.W.; Chapin, D.S.; Chen, Y.L.; DeMattos, S. B., et al., *ibid*, **1995**, *38*, 2802-2808.
7. Appleyard, M. E. *Trends Neurosci*. **1992**, *15*, 485-490.
8. Carson, K.A.; Guela, C.; Mesulam, M.M. *Brain Res*. **1991**, *540*, 204-208.
9. Tavitian, B.; Pappata, S.; Bonnot-Lours, S.; Prenant, C.; Jobert, A.; Crouzel, C.; DiGiamberardino, L. Euro. *J. Pharmacol*. **1993**, *236*, 229-238.
10.Tavitian, B.; Pappata, S.; Planas, A.M.; Jobert, A.; Bonnot-Lours, S.; Crouzel, C.; DiGiamberardino, L. *NeuroReport* **1993**, *4*, 535-538.
11. Planas, A.M.; Crouzel, C.; Hinnen, F.; Jobert, A.; Ne, F.; DiGiamberardino, L.; Tavitian, B. *Neuroimage* **1994**, *1*, 173-180.
12. Villalobos, A.; Biggers, C.K.; Blake, J.F.; Butler, T.W.; Chen, J.L. et al., Poster Presentation at the Annual Society of Neuroscience meeting, 1994.
13. Irie, T.; Fukushi, K.; Namba, H.; Iyo, M.; Nagatsuka, S. In: Abstracts of the Eleventh International Symposium on Radiopharmaceutical Chemistry, Vancouver, B.C. Canada, August, 1995, p. 458.
14. Mesulam, M.M.; Volicer, L.; Marquis, J.K.; Mufson, E.J.; Green, R.C. *Ann*. *Neurol*. **1986**, *19*, 144-151.
15. Games, D.; Adams, D.; Alessandrini, R.; Barbour, R.; Berthelette, P. et al. *Nature*, **1995**, *373*, 523-527.
16. Price, J.C.; Mayberg, H.S.; Dannals, R.F.; Wilson, A.A.; Raved, H.T. et at *J*. *Cereb. Blood Flow Metab*, **1993**, *13*, 656-667.
17. Ebmeier, K.P.; Hunter, R.; Curran, S.M.; Dougal, N.J.; Murray, C.L. et al. *Psychopharmacology*, **1992**, *108*, 103-109.

### SUMMARY OF THE INVENTION

This invention provides a method of detecting areas in a human brain which contain acetylcholinesterase comprising
a) injecting a positron-emitting radiolabeled compound of the formula 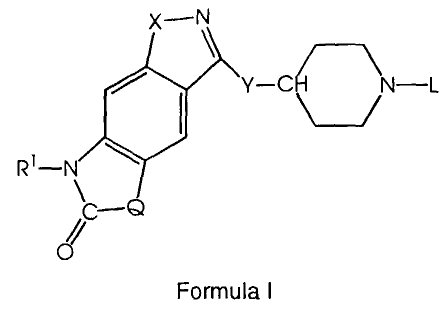 wherein
   Q is (CH₂)ₘ, -CH=CH-, -CHCH₃, -C(CH₃)₂, oxygen, sulfur or NH;
   X is oxygen or sulfur;
   Y is -(CH₂)ₘ-;
   L is phenyl or phenyl -(C₁-C₆)alkyl; wherein said phenyl is optionally substituted with (C₁-C₆)alkyl or halo, preferably I, F, or Br.
   R¹ is hydrogen or (C₁-C₆)alkyl;
   m is an integer from 1 to 3; and pharmaceutically acceptable salts thereof into the bloodstream of a human; and
b) imaging said brain of said human with positron emission tomography or single photon emission computerized tomography to produce a brain image, said image showing, the relative amounts of acetylcholinesterase in different portions of the brain.

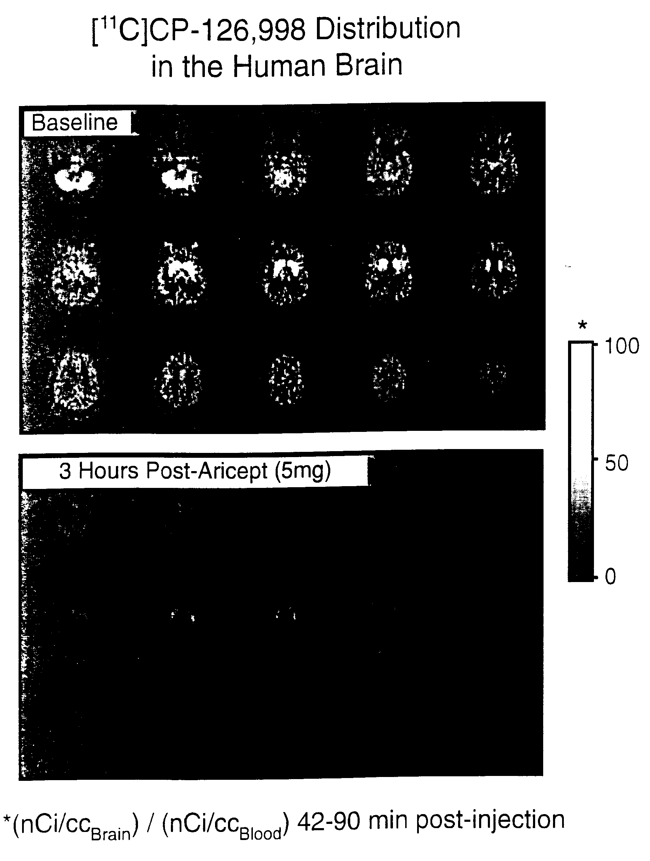

Preferred radiolabeled compounds of formula I for imaging brain acetylcholinesterase in Alzheimer's disease and other brain disorders are those wherein R³ is [¹¹C] methyl. Image is shown in Figure 1.

A second preferred group of compounds of formula I for imaging brain AChE are those wherein Y is ethylene and L is benzyl.

A still further preferred group of compounds of formula I for imaging brain AChE are those wherein X is -O-, Q is -CH₂-, and L is benzyl.

Another preferred group of compounds of formula I for imaging brain AChE are those wherein Q is -CH₂-, Y is ethylene, and L is benzyl.

A particularly preferred compound is 5,7-Dihydro-7-[¹¹C]methyl-3-[2-[1-(phenylmethyl)-4-piperidinyl]ethyl]-6H-pyrrolo[4,5-f]-1 ,2-benzisoxazole-6-one, the compound of Example 2.

In another preferred group L is benzyl in which the phenyl group is substituted with I or F.

The method of this invention may be used to diagnose the presence or severity or progression of Alzheimer's disease and other brain pathology.

### DETAILED DESCRIPTION OF THE INVENTION

An illustration of a method for synthesis of radiolabeled compounds of the present invention is set forth in Scheme 1 below.

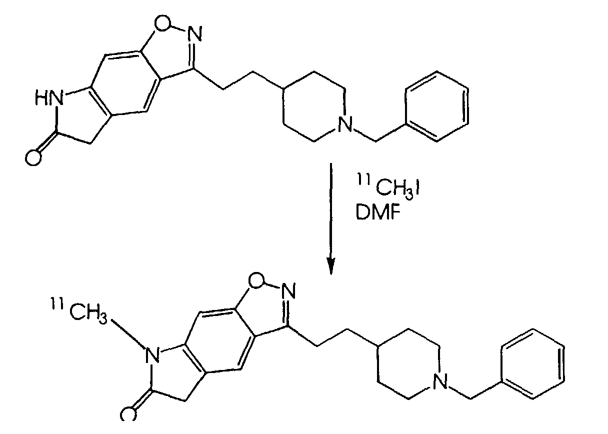

High specific activity [C-11] compound of Example 2 was synthesized (14-24% radiochemical yield, non-decay corrected) by treatment of the desmethyl precursor, compound of Example 1, with [C-11] methyl iodide and tetrabutylammonium hydroxide (TBAH) in DMF at 80°C for 5 min.

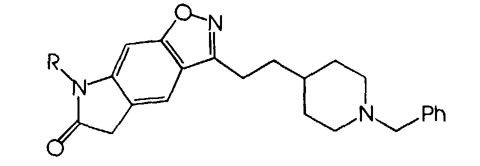

### R=[11C]Me, Example 2

### R=H, Example 1

The chemist of ordinary skill will recognize that synthetic procedures shown in WO 92/17475 for the compounds of formula I may be adapted to produce the corresponding radiolabeled compounds by indroducing radioactive intermediates at appropriate steps in the synthesis.

Preliminary in vivo studies with [C-11] compound of Example 2 in mice have been disclosed. (Musacher et al. J. Nuclear Med. 1996, 37(5), Supplement, Abstract No. 155).

Positron Emission Tomography (PET) is known in the art. See, for example, Maziere, *Pharmac. Ther*. 66, 83 (1995) who described *in vivo* use of PET to study cholinergic neurotrammone and Kilbourne, et al., *Synapse* 22, 123 (1996) who teach a method and modified equipment for primate imaging with PET.

Brain imaging has been greatly changed by the union of computer sciences with physics, thus creating many external detection systems. Among them, Positron Emission Tomography (PET) and Single Photon Emission Computerized Tomography (SPECT) can monitor non-invasively, using positron (β+) or γ cameras, the time-course of regional tissue radioactive concentration after administration of a compound labeled with a β+ or γ photon emitting radionuclide, respectively. When the labeled compound (radiopharmaceutical) is known to interact selectively and specifically with a known system of neurotransmission (transmitter, transporter, receptor, enzyme, etc.), then the radiopharmaceutical can be used as an *in vivo* probe to investigate the given neurotransmission system.

PET and SPECT methodologies allow *in vivo* sequential studies and radioactivity versus time can be plotted in selected regions of interest obtained with the serial images. These two methods are safe, non-invasive and due to the short half-life of the radioisotopes used, weakly irradiating. However, PET and SPECT have their own advantages and limits.

For PET studies, the main positron emitter radionuclides used for the labeling of cholinergic radiotracers are : Carbon 11 [¹¹C], having a 20.4 min half-life, Fluorine 18 [¹⁸F], with a 110 min half-life, and Bromine 76 [⁷⁶Br], with a 16 hr half-life. All of these radionuclides need to be prepared with very high specific activity in a cyclotron.

For SPECT studies, Iodine 123 [¹²³I] may be used for the study of the cholinergic system. Its half-life is 31.2 hr. This radioisotope is commercially available with very high specific activity.

In PET studies, as problems of attenuation and scatter corrections have been resolved, an absolute radiotracer quantitation in tissue is now possible in routine clinical application. However, the preparation of the positron emitter radionuclide (generally with a short half-life) is limited to facilities capable of producing the PET ligand.

Facilities which are capable of performing PET and SPECT imaging are available worldwide; a list of these facilities is published by ICP, Institute for Clinical PET. For example PET centers are located at Northern California PET Imaging Center, Sacramento, CA and Yale-VA Positron Imaging Laboratory, West Haven, CT.

The method of this invention provides methods of preparing radiolabeled compounds which bind strongly to AChE and are detectable by PET, producing an image of the human brain which shows the location and relative amount of AChE. Certain brain disorders, including Alzheimer's disease decrease the amount of AChE. Comparison of the brain image of test patient with normal brain images and images from patients known to have Alzheimer's disease shows the presence or severity of Alzheimer's disease in the test patient.

Choline acetyltransferase and AChE decrease significantly as plaque count rises and in demented subjects the reduction in choline acetyl transferase activity was found to correlate with intellectual impairment. Therefore there is a close relationship between changes in the cholinergic system and Alzheimer's disease. See Perry, et al., *Brit. Med. J*, 25, Nov. 1978, p. 1457.

The methods of the present invention are accomplished by administering to a patient suspected to be suffering from a brain disorder a non-toxic amount of a radiolabeled compound of formula I above. The amount of this compound must be sufficient to be detectable by PET techniques to produce a brain image. Decreased AChE as shown in brain image as compared with normal brain images is indicative of brain pathology in the patient.

### Example 1

### 5,7-Dihydro-3-[2-[1-(phenylmethyl)-4-piperidinyl]ethyl]-6H-pyrrolo [4,5-f]-1,2-benzisoxazol-6-one maleate

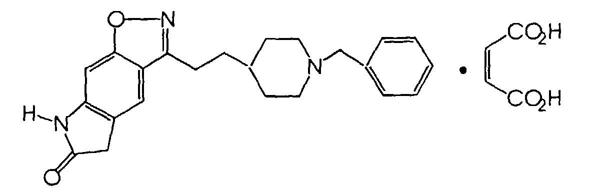

a) 5-Acetyl-1 ,3-dihydro-6-hydroxy-2 H-indol-2-one
   Acetyl chloride (4.09 mL, 0.0575 mol) was added to a slurry of aluminum trichloride (AlCl₃) (35.36 g, 0.265 mol) in carbon disulfide (CS₂) (250 mL). After 2-3 min, 6-methoxyoxindole (7.22 g, 0.0442 mol) was added. The resulting mixture was heated to reflux for 2.5 hours. (A black tar developed with time.) Excess solvent was decanted and ice water was added carefully to the residue. The resulting mixture was stirred overnight. The pale yellow solid obtained was collected, washed with water and dried under high vacuum to give the title compound (7.32 g, 87%).
   ¹H-NMR (DMSO-d₆) δ 13.0 (s, 1H), 10.8 (s, 1H), 7.70 (s, 1H), 6.30 (s, 1H), 3.40 (s, 2H), 2.54 (s, 3H).
b) 5-Acetyl-1 ,3-dihydro-6-hydroxy-2H-indol-2-one, 5-oxime
   An aqueous solution of hydroxylamine hydrochloride (8.26 g, 0.119 mol) and sodium acetate trihydrate (16.9 g, 0.124 mol) was added to a mixture of the ketone formed in step a (9.88 g, 0.0517 mol) in EtOH (600 mL). The resulting mixture was heated to reflux for 20 hours. The hot reaction mixture was filtered and the solid collected was rinsed with EtOH. After drying, the title compound (10.11 g, 95%) was obtained as a pale yellow solid).
   ¹H-NMR (DMSO-d₆) δ 12.0 (s, 1H), 11.4 (s, 1H), 10.5 (s, 1H), 7.29 (s, 1H), 6.35 (s, 1H), 3.38 (s, 2H), 2.20 (s, 3H).
c) 5-Acetyl-1 ,3-dihydro-6-hydroxy-2H-indol-2-one, 5-oxime acetate
   A heterogeneous mixture of the oxime formed in step b (7.15 g, 34.7 mmol) in acetic anhydride (55 mL) was heated at 80°C for 2 hours. The cooled reaction mixture was filtered and the solid collected was rinsed with water. After drying, the title compound (4.67 g, 54%) was obtained as a pale yellow solid.
   ¹H-NMR (DMSO-d₆) δ 11.3 (s, 1H), 10.6 (s, 1H), 7.35 (s, 1H), 6.44 (s, 1H), 3.41 (s, 2H), 2.37 (s, 3H), 2.21 (s, 3H).
d) 5,7-Dihydro-3-methyl-6H-pyrrolo[4,5-f]-1 ,2-benzisoxazol-6-one
   A mixture of the oxime acetate formed in step c (4.48 g, 18.0 mmol) and pyridine (14.6 mL, 180 mmol) in dimethylformamide (DMF) (660 mL) was heated at 125-130°C for 4 hours. The cooled reaction mixture was poured over water and extracted with EtOAc (4 times). The combined organic layer was washed with water and brine and dried (MgSO₄), filtered, and concentrated. Purification by silica gel flash chromatography (50% EtOAc/hexanes → 100% EtOAc) gave the title compound (2.20 g, 65%) as a pale yellow-orange solid.
   M.p. (EtOAc): 264-265°C (dec).
   ¹H-NMR (DMSO-d₆) δ 10.8 (s, 1H), 7.60 (s, 1H), 6.98 (s, 1H), 3.57 (s, 2H), 2.47 (s, 3H).
e) 4-[2-[5,7-Dihydro-6H-pyrrolo[4,5-f]-1 ,2-benzisoxazol-6-one-3-yl]ethyl]-1 - piperidinecarboxylic acid,1 -(1 ,1 -dimethylethyl)ester
   Freshly prepared 1 M Lithium diisopropyl amide (LDA) (40.9 mL, 40.9 mmol) was added dropwise and quickly to a cold (-78°C) solution of the benzisoxazole formed in step d (2.33 g, 12.4 mmol) in THF (400 mL). Immediately after addition was complete, a solution of 4-iodomethyl-1 -piperidinecarboxylic acid,1 -(1 ,1-dimethylethyl) ester (4.42 g, 13.6 mmol) in dry THF (100 mL) was added all at once. The resulting mixture was stirred at -78°C for 4 hours. Saturated ammonium chloride (NH₄Cl) was added and the mixture was extracted with ethyl acetate (EtOAc) (3 times). The combined organic layer was washed with brine, dried over magnesium sulfate (MgSO₄), filtered and concentrated. Purification by chromatography (20% → 30% EtOAc/CH₂Cl₂) gave recovered starting material (0.210 g, 9%) and the title compound (2.75 g, 58%) as an off-white solid.
   ¹H-NMR (CDCl₃) δ 8.48 (s, 1H), 7.44 (s, 1H), 7.03 (s, 1H), 4.08-4.14 (m, 2H), 3.63 (s, 2H), 2.97 (t, 2H, J=7.8 Hz), 2.69 (br t, 2H, J=12.8 Hz), 1.74-1.84 (m, 4H), 1.46-1.55 (m, 1H), 1.46 (s, 9H), 1.18 (ddd, 2H, J=24.4 Hz), J=12.1 Hz, J=4.3 Hz).
f) 5,7-Dihydro-3-[2-[1 -(phenylmethyl)-4-piperidinyl]ethyl]-6H-pyrrolo[4,5-f]-1 ,2-benzisoxazol-6-one maleate.

Trifluoroacetic acid (TFA (3.3 mL) was added dropwise to a cold (0°C) solution of the piperidine formed in step e (0.50 g, 1.30 mmol) in CH₂Cl₂ (13 mL). After 30 min, the mixture was concentrated and excess TFA was removed by concentrating from toluene (2 to 3 times). The crude residue was dissolved in DMF (12.5 mL) and sodium carbonate (Na₂CO₃) (0.689 g, 6.50 mmol) and benzyl bromide (0.186 mL, 1.56 mmol) were added. The resulting mixture was stirred at room temperature for 4 hours. The reaction was filtered and the filtrate was concentrated *in vacuo*. The residue was dissolved in methylene chloride and washed with brine, and dried (MGSO₄), filtered, and concentrated. Purification by silica gel chromatography (CH₂Cl₂ → 10% methanol/CH₂Cl₂) gave the title compound (free base) (0.343 g, 70%) as a white solid.

The maleate salt was prepared by adding a solution of maleic acid (0.061 g, 0.528 mmol) in ethanol (EtOH) (1 mL) to a solution of the free base (0.180 g, 0.48 mmol) in CH₂Cl₂ (10 mL). After concentrating, the salt was purified by recrystallization from isopropanol to give an off-white solid. Yield: 0.173 g, 73%.
M.p. 194-195°C.
   ¹H-NMR (DMSO-d₆) δ 10.82 (s, 1H), 7.65 (s, 1H), 7.48 (s, 5H), 7.00 (s, 1H), 6.03 (s, 1H), 4.24 (br s, 2H), 3.58 (s, 2H), 3.25-3.38 (m, 2H), 2.94 (t, 2H, J=7.6), 2.81 -2.97 (m, 2H), 1.86-1.96 (m, 2H), 1.62-1.76 (m, 2H), 1.30-1.60 (m, 3H).
Calc'd for C₂₃H₂₅N₃O₂·C₄H₄O₄: C, 65.97; H, 5.95; N, 8.55. Found: C, 65.98; H, 6.04; N, 8.54.

### Example 2

### 5,7-dihydro-7-[¹¹C]-methyl-3-[2-[1 -(phenylmethyl)-4-piperidinyl] ethyl] -6H-pyrrolo[4,5-f]-1,2-benzisoxazole-6-one

### Material and Methods

[¹¹C]carbon dioxide was produced by 16 MeV proton bombardment of a nitrogen gas target using a scanditronix RNP-16 biomedical cyclotron. Conversion to [¹¹C]methyl iodide has been described previously (I). Radioactivity measurements were made using a Capintec CRC-12 dose calibrator.

### Radiosynthesis, purification, and formulation of [¹¹C] 5,7-dihydro-7-[¹¹C]-methyl-3-[2-[1 -(phenylmethyl)-4-piperidinyl] ethyl]-6H-pyrrolo[4,5-f]-1 ,2-benzisoxazole-6-one

The maleate salt of the compound of example 1 (2 mg) was dissolved in water (0.5 mL) to which was added 2 pasteur pipet drops of 2N NaOH. The aqueous layer was extracted with diethyl ether (2 x 1 mL) and the extracts were passed through a Na₂SO₄ column (0.5 mm i.d. x 2.5 cm). The ether was evaporated under a gentle stream of argon. The white film was redissolved in 200 µL of DMF and transferred to a 1 mL septum sealed vial. The vial was cooled (-78°C) and [¹¹C]methyl iodide was passed into the reaction vessel by a stream of nitrogen carrier gas. When the radioactivity in the solution reached a plateau, the stream of nitrogen was stopped. Aqueous tetrabutylammonium hydroxide (5 µL, 0.4 M) was added to the reaction mixture via hamilton microsyringe. The reaction was heated in a 80°C water bath for 5 minutes prior to quenching by addition of 0.2 mL of HPLC solvent, consisting of 30:70 acetonitrile:water (0.1 M ammonium formate). The mixture was injected onto a Waters Nova-Pak C18 6µ (7.8 mm x 30 cm) semi-preparative column and eluted at a rate of 7 mL/min. The effluent from the column was monitored with a UV detector (254 nm, Waters module 440) and an in-line radioactivity detector (Ortec 449 ratemeter, 575 amplifier, 550 single channel analyzer, with a Nal (TI) cyystal). The radioactive peak corresponding to [¹¹C] the compound of example 2 (t_{R}= 5.2 min, *k*'=3.3) was collected in a rotary evaporator, and the solvent evaporated to dryness under reduced pressure. The residue was dissolved in sterile, normal saline (7 mL) and filtered through a sterile, 0.22 µm filter into a sterile, pyrogen-free evacuated vial. Sterile aqueous sodium bicarbonate (3 mL, 8.4%) was then added and the radioactivity was measured.

### Determination of specific radioactivity and radiochemical purity

An aliquot of the final solution of known volume and radioactivity was applied to an analytical reverse-phase HPLC cartridge (Waters Nova-Pak C18 6µ 8 mm x 1 cm radial compression cartridge). A mobile phase of 40:60 acetonitrile:water (0.1 M ammonium formate) with a flow rate of 4 mL/min was used to elute the radioligand (t_{R}=2.4 min. *k'*=2.4). The area of the UV absorbence peak measured at 254 nm corresponding to carrier product was measured by an automated integrating recorder (Hewlett Packard 3390A) and compared to a standard curve relating mass to UV absorbence.

The average radiochemical yield for the preparation of [¹¹C] compound of example 2 was 22% based on starting C-11 Mel (non-decay corrected, n=4). Average specific radioactivity was 1136 mCi/umol. Time of synthesis including formulation and specific radioactivity determination was approximately 25 minutes. In all instances [¹¹C] compound of example 2 proved to be of high radiochemical purity (>95%) and was found to be sterile and pyrogen-free.

Dannals R. F., Ravert H. T., Wilson A. A., and Wagner H. N. Jr, *Appl Radiat. Isot.* 37: 433 (1986).

### Example 3

### Imaging of Acetylcholinesterase in Human Brain

PET scans were used to image the brain distribution of [11C] CP-126,998 a highly selective AChE inhibitor in a 30 year-old normal healthy control. A thermoplastic mask was used for PET positioning and MRI was obtained for structural localization. A baseline and a blocking study approximately 3 hours after the oral administration of 5 mg of Donezepil Hydrochloride (Aricept), a reversible inhibitor of AchE, were obtained. Heated venous blood samples were obtained during both studies to measure the blood kinetics and metabolism.

Brain time activity curves show activity that plateaus from 42-90 minutes post-injection in the putamen, caudate nucleus and cerebellum. After normalization to the amount of circulating parent compound using the average ([nCi/ccBRAIN/nCi/ccPLASMA] from 42-90 minutes, the baseline rank order per region was: putamen (61), caudate (54), cerebellum (47), medulla oblongata (40), pons (36), thalamus (33), hippocampus (30) and frontal (26), temporal (26), parietal (27) and occipital cortex (23). The blocking study showed decrease binding in all brain regions: putamen (26), caudate (19), cerebellum (13) and frontal (7), parietal (7) cortex, a 58 to 72 percent reduction from the baseline study.

| **Uptake and displacement of [11-C]CP-126,998 in the brain of a single healthy volunteer subject** | | | |
|---|---|---|---|
| | Normalized uptake (tissue activity/plasma activity) | Normalized uptake post 5mg Aricept | Percent displacement by Aricept 5mg |
| **Putamen** | 61 | 26 | 57.4 |
| **Caudate** | 54 | 19 | 64.8 |
| **Cerebellum** | 47 | 13 | 72.3 |
| **Medulla Oblongata** | 40 | N/A | |
| **Pons** | 36 | N/A | |
| **Thalamus** | 33 | N/A | |
| **Hippocampus** | 30 | N/A | |
| **Frontal cortex** | 26 | 7 | 73.1 |
| **Temporal cortex** | 26 | N/A | |
| **Parietal cortex** | 27 | 7 | 74.1 |
| **Occipital cortex** | 23 | N/A | |

## Claims

1. A method of detecting areas in a human's brain which contain acetylcholin esterase comprising
a) injecting a detectable amount of a positron-emitting radiolabeled compound of the formula 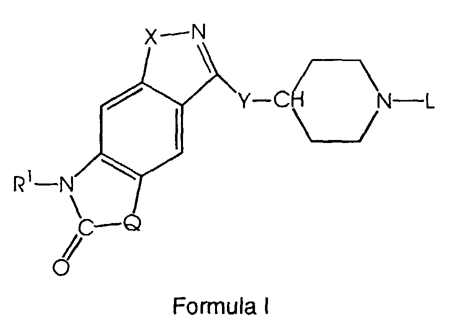 wherein
Q is (CH₂)ₘ, -CH=CH-, -CHCH₃, -C(CH₃)₂, oxygen, sulfur or NH;
X is oxygen or sulfur;
Y is (CH₂)ₘ-;
L is phenyl or phenyl -(C₁-C₆)alkyl; wherein said phenyl is optionally substituted with (C₁-C₆)alkyl or halo;
R¹ is hydrogen or (C₁-C₆)alkyl;
m is an integer from 1 to 3; and pharmaceutically acceptable salts thereof; into the bloodstream of a human; and
b) imaging said brain of said human with positron emission tomography or single photon emission computerized tomography to produce a brain image, said image showing, the relative amounts of acetylcholinesterase in different portions of the brain.

2. A method of claim 1 wherein R¹ is [¹¹C] methyl.

3. A method of claim 2 wherein Y is ethylene and L is benzyl.

4. A method of claim 2 wherein X is -O-; Q is -CH₂-; and L is benzyl.

5. A method of claim 4 wherein Y is ethylene.

6. The method of claim 1 wherein said positron-emitting radiolabeled compound is 5,7-dihydro-7-[¹¹C]-methyl-3-[2-[1 -(phenylmethyl)-4-piperidinyl]ethyl]-6H-pyrrolo[4,5-f]-1 ,2-benzisoxazole-6-one.

7. A method of diagnosing Alzheimer's disease, establishing disease severity and following the course or progression of said disease comprising
a) injecting a detectable amount of a positron-emitting radiolabeled compound of the formula 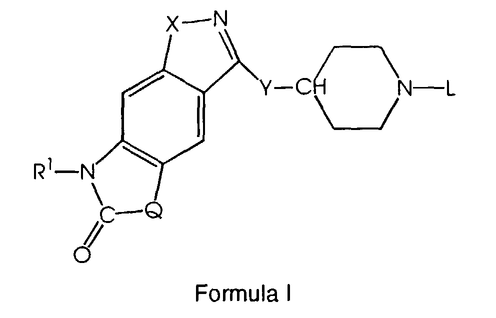 wherein
Q is (CH₂)ₘ, -CH=CH-, -CHCH₃, -C(CH₃)₂, oxygen, sulfur or NH;
X is oxygen or sulfur;
Y is -(CH₂)ₘ-;
L is phenyl or phenyl -(C₁-C₆)alkyl; wherein said phenyl is optionally substituted with (C₁-C₆)alkyl or halo;
R¹ is hydrogen or (C₁-C₆)alkyl;
m is an integer from 1 to 3; and pharmaceutically acceptable salts thereof; into the bloodstream of a human; and
b) imaging said brain of said human with positron emission tomography or single photon emission computerized tomography to produce a brain image; and
c) relating said brain image to the presence or absence or degree of severity or progression of said Alzheimer's disease.

8. A method of claim 7 wherein R³ is [¹¹C] methyl.

9. A method of claim 8 wherein Y is ethylene and L is benzyl.

10. A method of claim 8 wherein X is -O-; Q is -CH₂-; and L is benzyl.

11. A method of claim 10 wherein Y is ethylene.

12. The method of claim 7 wherein said positron-emitting radiolabeled compound is 5,7-dihydro-7-[¹¹C]-methyl-3-[2-[1 -(phenylmethyl)-4-piperidinyl]ethyl]-6H-pyrrolo[4,5-f]-1 ,2-benzisoxazole-6-one.
